# EUROPEAN PATENT APPLICATION

(11) **EP 4 379 300 A1**
(43) Date of publication of application: **05.06.2024**
(21) Application number: 22848155.2
(22) Date of filing: 27.06.2022
(51) Int. Cl.: F25D 23/12, F25D 31/00, A47J 43/046, A47J 43/08, A47J 43/07, B08B 3/12, A61L 2/025

(54) **REFRIGERATOR, AND CONTROL METHOD FOR ULTRASONIC-ASSISTED PROCESSING APPARATUS THEREOF**

(30) Priority: 30.07.2021 CN 202110875175
(71) Applicant: Qingdao Haier Refrigerator Co., Ltd., Laoshan District Qingdao Shandong 266101 (CN); Haier Smart Home Co., Ltd., Qingdao, Shandong 266101 (CN)
(72) Inventor: ZHAO, Bintang, Qingdao, Shandong 266101 (CN); WANG, Liyan, Qingdao, Shandong 266101 (CN); WANG, Jing, Qingdao, Shandong 266101 (CN); FEI, Bin, Qingdao, Shandong 266101 (CN)
(74) Representative: Winter, Brandl - Partnerschaft mbB
(86) International application number: PCT/CN2022/101561
(87) International publication number: WO 2023/005548

(57) **Abstract**

Provided are a refrigerator and a control method for an ultrasound-assisted processing device thereof. The control method includes: detecting an ultrasonic container using a signal sensing device on an ultrasonic base; determining a function type of the ultrasonic container based on a detection result; acquiring ultrasonic parameters corresponding to the function type of the ultrasonic container; controlling the ultrasound-assisted processing device to operate according to the ultrasonic parameters. The present invention is capable of realizing the purpose that the ultrasound-assisted processing devices has multiple functions and purposes.

## Description

### TECHNICAL FIELD

The present invention relates to the technical field of refrigerators, and in particular to a refrigerator and a control method for an ultrasound-assisted processing device thereof.

### BACKGROUND ART

Currently, the evaluation of a refrigerator for storage performance is no longer a simple extension of a storage time. Some users have also demanded the realization of certain auxiliary functions (such as auxiliary pickling, auxiliary cleaning, etc.).

In the prior art, numerous improvement schemes have emerged. Among them, the most common is the addition of an ultrasonic equipment in a storage compartment of the refrigerator, using the ultrasonic equipment for auxiliary processing work, thereby improving the processing effect and efficiency of ingredients.

However, since the inherent frequency of the ultrasonic transducer is fixed and cannot be changed through software, a set of ultrasonic equipment has a single purpose. For example, an ultrasonic cleaner is only suitable for cleaning and cannot be used for other purposes that require changing frequency. If one wants to achieve ultrasonic-assisted pickling, a new set of equipment is needed, which is not conducive to achieve the purpose of multifunctionality and multipurpose use of the ultrasonic equipment. Therefore, improvements are still needed.

### SUMMARY OF THE INVENTION

In a first aspect, an objective of the present invention is to enable the ultrasound-assisted processing device to have multiple functions and purposes.

In the first aspect, a further objective of the present invention is to ensure the processing effect on ingredients.

In a second aspect, an objective of the present invention is to provide a refrigerator.

Particularly, according to the first aspect of the present invention, the present invention provides a control method for an ultrasound-assisted processing device inside a refrigerator, including:
detecting an ultrasonic container using a signal sensing device on an ultrasonic base;
determining a function type of the ultrasonic container based on a detection result;
acquiring ultrasonic parameters corresponding to the function type of the ultrasonic container;
controlling the ultrasound-assisted processing device to operate according to the ultrasonic parameters.

Alternatively, the function type of the ultrasonic container includes:
ultrasonic-assisted pickling, ultrasonic-assisted cleaning, ultrasonic-assisted atomizing.

Alternatively, the ultrasonic parameters include a frequency of the ultrasonic generator;
the step of acquiring ultrasonic parameters corresponding to the function type of the ultrasonic container includes:
acquiring a frequency of the ultrasonic transducer corresponding to the function type of the ultrasonic container, and determining a frequency of the ultrasonic generator based on the frequency of the ultrasonic transducer.

Alternatively, the ultrasonic parameters also include a power of the ultrasonic generator;
the step of acquiring ultrasonic parameters corresponding to the function type of the ultrasonic container also includes:
acquiring the power of the ultrasonic generator corresponding to the function type of the ultrasonic container.

Alternatively, the ultrasonic parameters also include a target duty cycle;
the step of acquiring ultrasonic parameters corresponding to the function type of the ultrasonic container also includes:
acquiring the target duty cycle corresponding to the function type of the ultrasonic container.

Alternatively, before detecting the ultrasonic container using the signal sensing device on the ultrasonic base, it also includes:
determining whether an ultrasonic container is placed on the ultrasonic base, if so, then detecting the ultrasonic container using the signal sensing device on the ultrasonic base.

Alternatively, after controlling the ultrasound-assisted processing device to operate according to the ultrasonic parameters, it also includes:
determining whether a total processing time of the ultrasound-assisted processing device has reached a preset processing time, if so, then turning off the ultrasound-assisted processing device.

According to the second aspect of the present invention, a refrigerator is provided. The refrigerator includes:
an ultrasound-assisted processing device, including an ultrasonic base, at least one ultrasonic container for use with the ultrasonic base, and a signal sensing device for detecting the function type of the ultrasonic container;
a controller, including a memory and a processor, wherein a control program is stored in the memory, and the control program, when executed by the processor, is configured to implement any one of the control methods for the ultrasound-assisted processing device described above.

Alternatively, the signal sensing device includes two Hall sensors located on the ultrasonic base, a bottom of the ultrasonic container has a characteristic part, which is configured to be detected and recognized by the two Hall sensors, thereby determining the function type of the ultrasonic container.

Alternatively, an outer shell of the ultrasonic container is provided with a label indicating the function type.

In the control method for the ultrasound-assisted processing device according to the present invention, since a base is provided with the signal sensing device, the signal sensing device can automatically recognize ultrasonic containers of different function types. This allows for the acquisition of different ultrasonic parameters corresponding to the different function types of the ultrasonic containers, and controls the ultrasound-assisted processing device to operate according to the ultrasonic parameters. This achieves the automatic recognition of the function type of ultrasonic containers and can adjust the ultrasonic parameters automatically based on the function type, making the functionality of the ultrasound-assisted processing device more versatile.

Further, the refrigerator of the present invention and the control method of the ultrasound-assisted processing device thereof, by accumulating the total processing time of the ultrasound-assisted processing device and turning off the ultrasound-assisted processing device when the total processing time of the ultrasound-assisted processing device reaches a preset processing time, can avoid overprocessing of the ingredients and ensure the quality of the processing of the ingredients.

The aforesaid and other objectives, advantages and features of the present invention will be more apparent to those skilled in the art from the following detailed description of the specific embodiments of the present invention with reference to the accompanying drawings.

### BRIEF DESCRIPTION OF THE DRAWINGS

Various other advantages and benefits will be apparent to those of ordinary skill in the art by reading detailed descriptions of the preferred embodiments below. The accompanying drawings are only for the purpose of illustrating the preferred embodiments and shall not be construed as limitations to the present invention. In addition, the same reference numbers are used to represent the same components throughout the drawings, in which:
FIG. 1 is a schematic structural diagram of a refrigerator according to an embodiment of the present invention;
FIG. 2 is a schematic structural diagram of an ultrasonic base according to an embodiment of the present invention;
FIG. 3 is a schematic structural diagram of an ultrasonic container according to an embodiment of the present invention;
FIG. 4 is a schematic block diagram of the refrigerator according to the embodiment of the present invention;
FIG. 5 is a schematic diagram of a control method for an ultrasound-assisted processing device according to an embodiment of the present invention;
FIG. 6 is a flowchart of the control method for the ultrasound-assisted processing device according to the embodiment of the present invention.

### DETAILED DESCRIPTION

Exemplary embodiments of the present disclosure will be described in more detail below with reference to the accompanying drawings. However, although the exemplary embodiments of the present disclosure are shown in the drawings, it should be understood that the present disclosure may be implemented in various ways and shall not be limited by the embodiments set forth herein. On the contrary, these embodiments are provided for more thorough understanding of the present disclosure, and can fully convey the scope of the present disclosure to those skilled in the art.

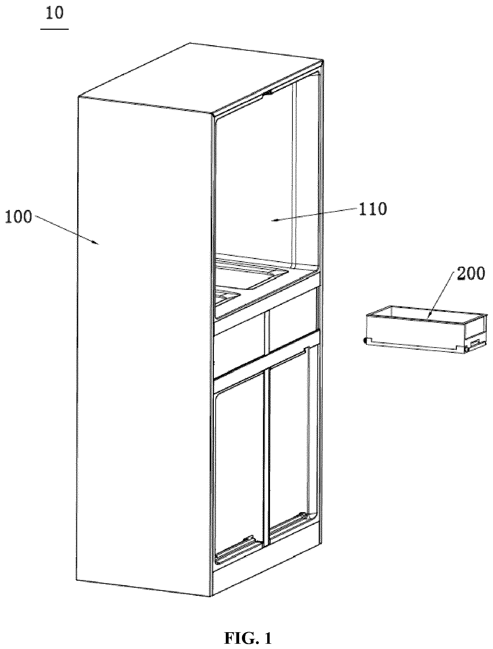

FIG. 1 is a schematic structural diagram of a refrigerator 10 according to an embodiment of the present invention. The refrigerator 10 may generally include a cabinet 100, and one or more storage compartments 110 are formed in the cabinet 100. The storage compartments 110 may be configured to form a refrigerated storage space 110, a frozen storage space 110, a variable temperature storage space 110 and the like according to refrigerating temperatures. Specifically, the number, functions and layout of the storage compartments 110 may be configured as required.

According to the present invention, the refrigerator 10 may further include an ultrasound-assisted processing device 200 (not shown in FIG. 1). The ultrasound-assisted processing device is located within the refrigerated storage space 110 of the refrigerator 10. Of course, in other cases, the ultrasound-assisted processing device can also be located within the frozen storage space 110 or the variable temperature storage space 110. The functions of the ultrasound-assisted processing device include, but are not limited to, assisting in pickling, cleaning, atomizing and the like. The ultrasound-assisted processing device can be mounted in the form of a drawer in the refrigerated storage space 110 or directly placed in the refrigerated storage space 110.

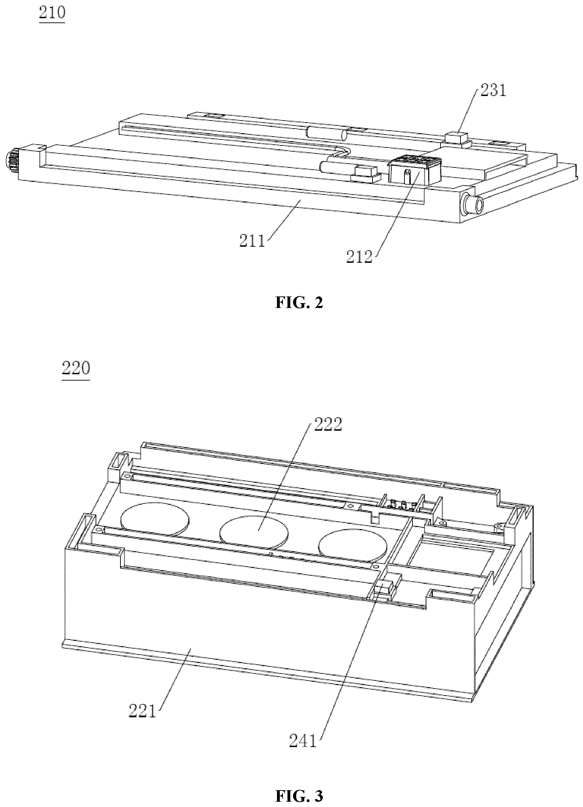

FIG. 2 is a schematic structural diagram of an ultrasonic base according to an embodiment of the present invention; In this embodiment, the ultrasound-assisted processing device includes an ultrasonic base 210, at least one ultrasonic container 220 for use with the ultrasonic base 210, and a signal sensing device 230 for detecting the function type of the ultrasonic container. In this embodiment, the ultrasonic base is placed inside the storage compartment, and there can be three ultrasonic containers for use with the ultrasonic base, which can be defined as an ultrasonic pickling container, an ultrasonic cleaning container, and an ultrasonic atomizing container, respectively.

To make it easier for users to identify different functional types of the ultrasonic containers, labels can be placed on outer shells of the ultrasonic containers. For example, a label with the word "pickling" can be affixed to the outer shell of the ultrasonic pickling container, a label with the word "cleaning" can be affixed to the outer shell of the ultrasonic cleaning container, and a label with the word "atomizing" can be affixed to the outer shell of the ultrasonic atomizing container. If a user wants to pickle ingredients, they can easily find the ultrasonic pickling container through the label and then place the ultrasonic pickling container on the ultrasonic base separately. Similarly, if a user wants to clean or atomize ingredients, they can find the respective ultrasonic cleaning container or ultrasonic atomizing container through the labels and place them on the ultrasonic base. It should be noted that the ultrasonic base can only accommodate one function type of the ultrasonic container at a time.

As mentioned before, since the frequency of the ultrasonic transducer is fixed (inherent frequency) and cannot be changed through software, a set of ultrasonic equipment can only engage in one type of processing work. For example, an ultrasonic cleaning equipment can only be used for cleaning ingredients and not for other purposes. If one wants to pickle ingredients ultrasonically, a new set of equipment is needed. To address this issue, the present invention proposes a solution of an ultrasonic generator matching multiple ultrasonic transducers, thus realizing the multifunctional and multipurpose switching of the ultrasonic device within the refrigerator 10.

Specifically, the ultrasonic container 220 includes a container body 221 and an ultrasonic transducer 222, and the ultrasonic transducer is fixed at the bottom of the container body. The ultrasonic base 210 includes a base body 211 and an ultrasonic generator 212, and the ultrasonic generator is fixed at the top of the base body. By designing the structure of the ultrasonic container and the ultrasonic base accordingly, when the ultrasonic container is placed on the ultrasonic base, a circuit between the ultrasonic generator and the ultrasonic transducer can be directly connected. A drive signal produced by the ultrasonic generator can be transmitted through the circuit to the ultrasonic transducer, driving the ultrasonic transducer to vibrate.

In a preferred embodiment, the signal sensing device 230 includes two Hall sensors 231, which are embedded at the top of the base body, with their probes protruding above the upper surface of the base body. A bottom of the ultrasonic container is equipped with a characteristic part designed to be detected and recognized by the two Hall sensors, thereby determining the function type of the ultrasonic container.

In a specific implementation, the two Hall sensors are oppositely positioned on both sides of the base body along its length direction. The characteristic part of the container body can be indicated by whether magnets 241 are installed. For example, one side along the length direction of the container body can be defined as a first side, and the other side along the length direction of the container body can be defined as a second side. For the ultrasonic pickling container, its characteristic part could have no magnets on both sides, so when the ultrasonic pickling container is placed on the ultrasonic base, both Hall sensors would output a low voltage, which can be represented as (0,0). For the ultrasonic cleaning container, its characteristic part could have no magnet near the first side of the container body and a magnet near the second side, so when the ultrasonic cleaning container is placed on the ultrasonic base, the Hall sensor near the first side of the container body would output a low voltage, and the Hall sensor near the second side would output a high voltage, which can be represented as (0,1). For the ultrasonic atomizing container, its characteristic part could have a magnet near the first side of the container body and no magnet near the second side, so when the ultrasonic atomizing container is placed on the ultrasonic base, the Hall sensor near the first side would output a high voltage, and the Hall sensor near the second side would output a low voltage, which can be represented as (1,0). This allows for a convenient and straightforward distinction of the function type of the ultrasonic containers.

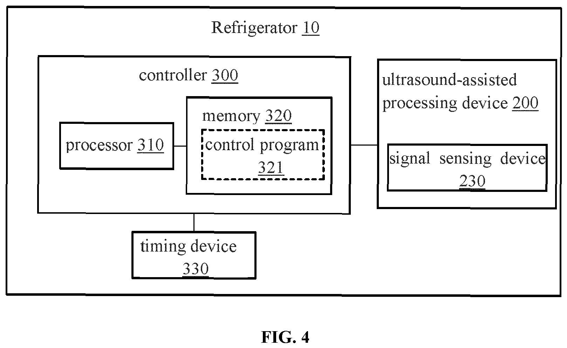

FIG. 4 is a schematic block diagram of the refrigerator according to the embodiment of the present invention. Furthermore, the refrigerator 10 can also be equipped with a controller 300 and a timing device 330 and the like.

The controller 300 includes a memory 320 and a processor 310, and a control program 321 is stored in the memory 320. The control program, when executed by the processor 310, is configured to implement a control method for the ultrasound-assisted processing device 200 according to this embodiment. The controller is in signal connection with the ultrasonic generator and the signal sensing device, providing control signals to the ultrasonic generator based on the detection results of the signal sensing device, thus adjusting the power and frequency of the ultrasonic generator, as well as starting and stopping the ultrasonic generator. The controller may be integrated onto a main control panel of the refrigerator 10, or may be separately arranged adjacent to the ultrasonic generator. The controller may further be in signal connection with a main control device of the refrigerator 10 to provide the main control device with a running state of the ultrasonic generator and to receive a control command from the main control device.

The controller may be implemented by various devices with certain data processing capabilities. In a typical configuration, the controller may include a memory, a processor, an input/output interface, and the like.

The timing device can be a timer, used to accumulate a total processing time of the ultrasound-assisted processing device, in order to turn off the ultrasound-assisted processing device after the total processing time reaches a preset processing time, preventing the failure of ingredient processing due to overprocessing.

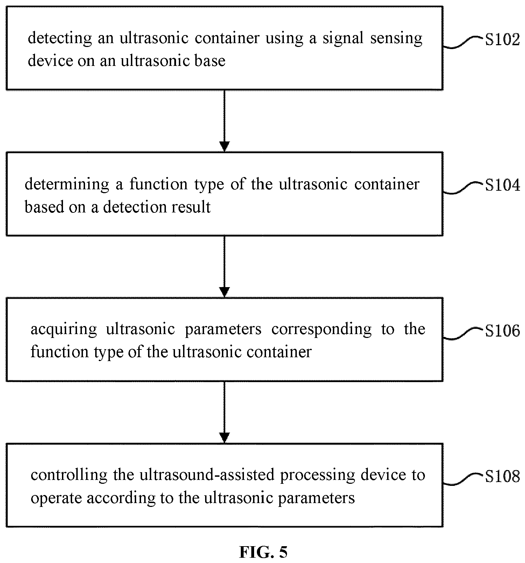

FIG. 5 is a schematic diagram of a control method for an ultrasound-assisted processing device according to an embodiment of the present invention. The control method at least includes steps S 102 to S 108 as follows.

In step S102, detecting the ultrasonic container using the signal sensing device on the ultrasonic base.

In step S 104, determining the function type of the ultrasonic container based on a detection result.

In this step, it should be noted that there can be multiple ultrasonic containers used in conjunction with the ultrasonic base, each ultrasonic container has a different function type, to facilitate different food processing functions.

In step S106, acquiring ultrasonic parameters corresponding to the function type of the ultrasonic container.

In step S 108, controling the ultrasound-assisted processing device to operate according to the ultrasonic parameters.

In the control method for the ultrasound-assisted processing device 200 according to the present invention, since a base is provided with the signal sensing device, the signal sensing device can automatically recognize ultrasonic containers of different function types. This allows for the acquisition of different ultrasonic parameters corresponding to the different function types of the ultrasonic containers, and controls the ultrasound-assisted processing device to operate according to the ultrasonic parameters. This achieves the automatic recognition of the function type of ultrasonic containers and can adjust the ultrasonic parameters automatically based on the function type, making the functionality of the ultrasound-assisted processing device more versatile.

In this embodiment, the function types of the ultrasonic container include, but are not limited to, ultrasonic-assisted pickling, ultrasonic-assisted cleaning, ultrasonic-assisted atomization, and for example, ultrasonic-assisted freezing.

The ultrasonic parameters include the frequency of the ultrasonic generator. The step of acquiring the ultrasonic parameters corresponding to the function type of the ultrasonic container may involve obtaining a frequency of the ultrasonic transducer corresponding to the function type of the ultrasonic container, and determining a frequency of the ultrasonic generator based on the frequency of the ultrasonic transducer. Since the frequency of the ultrasonic transducers at the bottom of the ultrasonic containers varies according to their function types, to send a matching drive signal from the ultrasonic generator to the ultrasonic transducer, it is necessary to ensure that a power of the ultrasonic generator matches the frequency of the ultrasonic transducer.

Furthermore, the ultrasonic parameters also include the power of the ultrasonic generator. The step of acquiring the ultrasonic parameters corresponding to the function type of the ultrasonic container also includes obtaining a power of the ultrasonic generator corresponding to the function type of the ultrasonic container. For example, when using the ultrasonic container for cleaning ingredients, the power of the ultrasonic generator is relatively high to improve cleaning speed. When using the ultrasonic container for pickling ingredients, the power of the ultrasonic generator is relatively low to ensure the effectiveness of pickle.

Moreover, the ultrasonic parameters can also include a target duty cycle. The step of acquiring the ultrasonic parameters corresponding to the function type of the ultrasonic container also involves obtaining the target duty cycle corresponding to the function type of the ultrasonic container. Continuous vibration of the ultrasonic transducer generates a significant amount of heat, and when the heat is transferred to the container body, it would cause the temperature during ingredient processing to become too high easily. By controlling the ultrasound-assisted processing device to operate according to the target duty cycle, the ultrasound-assisted processing device can cool down in a timely manner, thus preventing the container body's temperature from becoming too high.

Considering that when an ultrasonic pickling container is placed on the base and when no ultrasonic container is placed on the base, the output from the two Hall sensors is the same low level, i.e., (0,0). Therefore, to avoid mistakenly identifying the absence of an ultrasonic container on the base as the presence of an ultrasonic pickling container, this embodiment includes a step before detecting the ultrasonic container with the signal sensing device on the ultrasonic base: determining whether an ultrasonic container is placed on the ultrasonic base. Once the placement of the ultrasonic container is confirmed, the detection of the ultrasonic container using the signal sensing device on the ultrasonic base is performed.

Since different ingredients require different preset processing times, for example, chicken needs 30 minutes, pork needs 40 minutes, and beef requires 60 minutes, this embodiment includes a step after controlling the ultrasound-assisted processing device to operate according to the ultrasonic parameters on a cyclical basis: determining whether the total processing time of the ultrasound-assisted processing device has reached the preset processing time. If so, the ultrasound-assisted processing device is turned off; if not, the ultrasound-assisted processing device continues to operate.

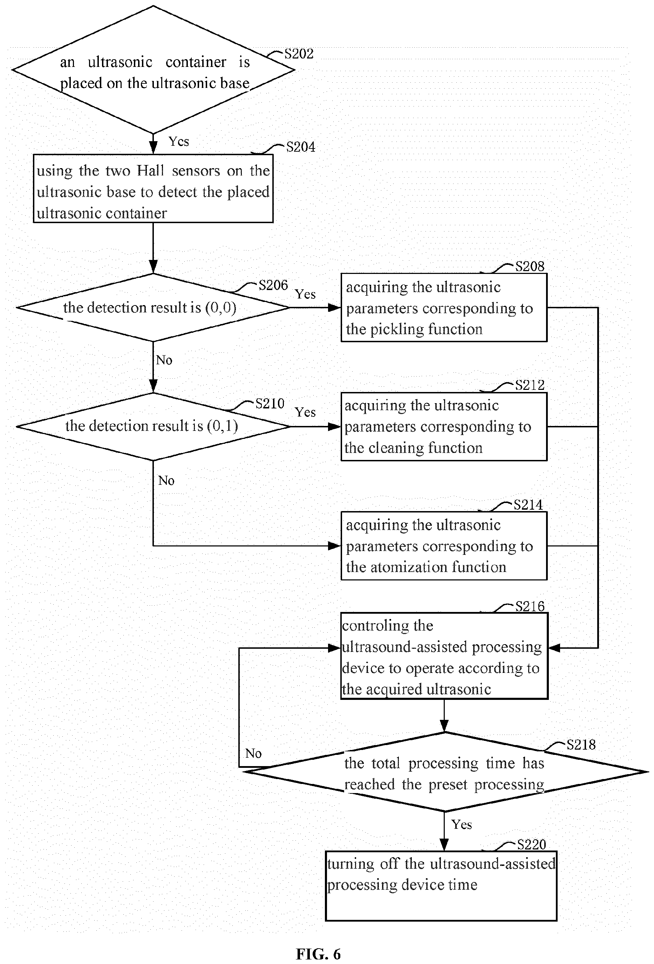

FIG. 6 is a flowchart of the control method for the ultrasound-assisted processing device according to the embodiment of the present invention. The control method at least includes steps S 102 to S 108 as follows for the ultrasonic generator:
In step S202, determining whether an ultrasonic container is placed on the ultrasonic base. If so, proceed to step S204.

In step S204, using the two Hall sensors on the ultrasonic base to detect the placed ultrasonic container.

In step S206, determining if the detection results of the two Hall sensors are (0,0). If so, proceed to step S208; if not, proceed to step S210.

In step S208, acquiring the ultrasonic parameters corresponding to the pickling function, then proceed to step S216.

In step S210, determining if the detection results of the two Hall sensors are (0,1). If so, proceed to step S212; if not, proceed to step S214.

In step S212, acquiring the ultrasonic parameters corresponding to the cleaning function, then proceed to step S216.

In step S214, acquiring the ultrasonic parameters corresponding to the atomization function, then proceed to step S216.

In step S216, controling the ultrasound-assisted processing device to operate according to the acquired ultrasonic parameters.

In step S218, determining if the total processing time of the ultrasound-assisted processing device has reached the preset processing time. If so, proceed to step S220; if not, return to step S216.

In step S220, turning off the ultrasound-assisted processing device.

Therefore, those skilled in the art should appreciate that although multiple exemplary embodiments of the present invention have been illustrated and described in detail, many other variations or modifications that accord with the principle of the present invention may be still determined or derived directly from the content disclosed by the present invention without departing from the spirit and scope of the present invention. Thus, the scope of the present invention should be understood and deemed to include all these and other variations or modifications.

## Claims

1. A control method for an ultrasound-assisted processing device, comprising:
detecting an ultrasonic container using a signal sensing device on an ultrasonic base;
determining a function type of the ultrasonic container based on a detection result;
acquiring ultrasonic parameters corresponding to the function type of the ultrasonic container;
controlling the ultrasound-assisted processing device to operate according to the ultrasonic parameters.

2. The control method for the ultrasound-assisted processing device according to claim 1, wherein the function type of the ultrasonic container comprises:
ultrasonic-assisted pickling, ultrasonic-assisted cleaning, ultrasonic-assisted atomizing.

3. The control method for the ultrasound-assisted processing device according to claim 1 or 2, wherein:
the ultrasonic parameters comprise a frequency of the ultrasonic generator;
the step of acquiring ultrasonic parameters corresponding to the function type of the ultrasonic container comprises:
acquiring a frequency of the ultrasonic transducer corresponding to the function type of the ultrasonic container, and determining a frequency of the ultrasonic generator based on the frequency of the ultrasonic transducer.

4. The control method for the ultrasound-assisted processing device according to claim 3, wherein:
the ultrasonic parameters also comprise a power of the ultrasonic generator;
the step of acquiring ultrasonic parameters corresponding to the function type of the ultrasonic container also comprises:
acquiring the power of the ultrasonic generator corresponding to the function type of the ultrasonic container.

5. The control method for the ultrasound-assisted processing device according to claim 4, wherein:
the ultrasonic parameters also comprise a target duty cycle;
the step of acquiring ultrasonic parameters corresponding to the function type of the ultrasonic container also comprises:
acquiring the target duty cycle corresponding to the function type of the ultrasonic container.

6. The control method for the ultrasound-assisted processing device according to claim 1 or 2, wherein, before detecting the ultrasonic container using the signal sensing device on the ultrasonic base, it also comprises:
determining whether an ultrasonic container is placed on the ultrasonic base, if so, then detecting the ultrasonic container using the signal sensing device on the ultrasonic base.

7. The control method for the ultrasound-assisted processing device according to claim 1 or 2, wherein, after controlling the ultrasound-assisted processing device to operate according to the ultrasonic parameters, it also comprises:
determining whether a total processing time of the ultrasound-assisted processing device has reached a preset processing time, if so, then turning off the ultrasound-assisted processing device.

8. A refrigerator, comprising:
an ultrasound-assisted processing device, comprising an ultrasonic base, at least one ultrasonic container for use with the ultrasonic base, and a signal sensing device for detecting the function type of the ultrasonic container;
a controller, comprising a memory and a processor, wherein a control program is stored in the memory, and the control program, when executed by the processor, is configured to implement the control method for the ultrasound-assisted processing device according to any one of claims 1 to 7.

9. The refrigerator according to claim 8, wherein:
the signal sensing device comprises two Hall sensors located on the ultrasonic base, a bottom of the ultrasonic container has a characteristic part, which is configured to be detected and recognized by the two Hall sensors, thereby determining the function type of the ultrasonic container.

10. The refrigerator according to claim 8, wherein:
an outer shell of the ultrasonic container is provided with a label indicating the function type.
